(19) Europäisches Patentamt
European Patent Office
Office européen des brevets

(11) Publication number: 0 445 062 A1

(12) **EUROPEAN PATENT APPLICATION**

(21) Application number: 91500018.6

(51) Int. Cl.[5]: C07D 215/38, A61K 31/47

(22) Date of filing: 15.02.91

(30) Priority: 26.02.90 GB 9004288

(43) Date of publication of application: 04.09.91 Bulletin 91/36

(84) Designated Contracting States: AT BE CH DE DK ES FR GB GR IT LI LU NL SE

(71) Applicant: LABORATORIOS ALMIRALL SA
Cardoner 68-74
E-08024 Barcelona (ES)

(72) Inventor: Duran, Carlos Puig
calle Mayor de Sarria, 210 entlo. 2a
E-08017 Barcelona (ES)
Inventor: Noguera, Fernando Pujol
Graus 2, 72, 2e, 4e
Barcelona (ES)
Inventor: Soto, Jose Manuel Prieto
Rabassa 46-48, 2e, 2e, Esc. B.
Barcelona-24 (ES)
Inventor: Noverola, Armando Vega
Traversera de Dalt, 62-64, 7e, 3e, 2e Esc. B.
Barcelona-24 (ES)
Inventor: Gristwood, Robert W.
Calle Granollers 55B
San Cugat del Valles, Barcelona (ES)

(74) Representative: Goldin, Douglas Michael et al
J.A. KEMP & CO. 14, South Square Gray's Inn
London WC1R 5EU (GB)

# (54) 4(1H)Quinolone derivatives.

(57) The invention provides 4(1H)quinolone derivatives of the formula I:

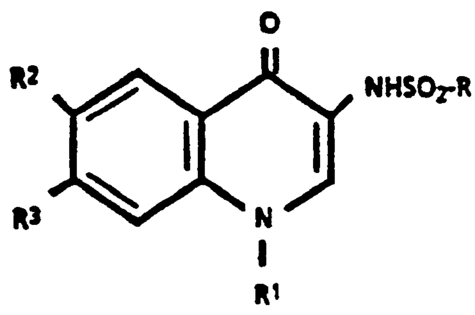

I

wherein R represents an alkyl group containing up to 6 carbon atoms,
$R^1$ represents an alkyl or alkenyl group containing up to 6 carbon atoms,
$R^2$ and $R^3$ singly each represents hydrogen, halogen, methoxy or a trifluoromethyl group, with the proviso that at least one of $R^2$ and $R^3$ is hydrogen, and pharmaceutically acceptable salts thereof and processes for their production. The compounds are useful for the treatment of cardiovascular and respiratory disorders.

EP 0 445 062 A1

# 4(1H)QUINOLONE DERIVATIVES

This invention relates to new therapeutically useful 4(1H)quinolone derivatives, to process for their preparation and pharmaceutical compositions containing them.

3-Substituted-4(1H)quinolones are disclosed in Belgian Patents Ns. 882443 and 890477 and Eur. Pat. Appl. 172004, and showed antihypertensive activity. Among these compounds,7-fluoro-1-methyl-3-methylsulfinyl-4(1H)quinolone (Flosequinan, INN) is of outstanding interest. On the other hand, T.Itai et al. (Chemical Abstracts Vol. 71, 113122 a (1969)) disclosed 1-methyl-3-amino-4(1H)quinolone which had no appreciable pharmacological activities.

In a publication about antibacterial quinolones (R. Krishnan et al. J. Het. Chem. 23, 1801 (1986)), the compound of formula A has been disclosed.

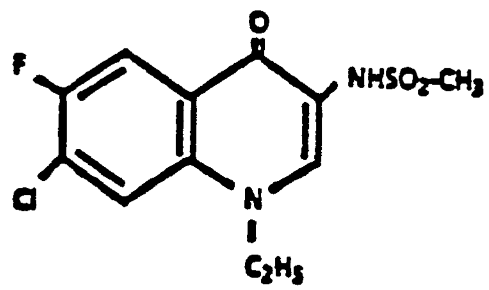

A

This does not exhibit any interesting antibacterial activity.

We have now surprisingly found that 4(1H)quinolone derivatives with a substituent in 6 or 7 position and an alkylsulfonamido group in position 3, provide new compounds that present not only a higher, oral and longer lasting antihypertensive activity, but a lower toxicity.

Consequently the new 4(1H)quinolone derivatives of the invention represent an important advance over Flosequinan and the related compounds described in the above documents.

Accordingly, the present invention provides a 4(1H)quinolone derivative of the formula I:

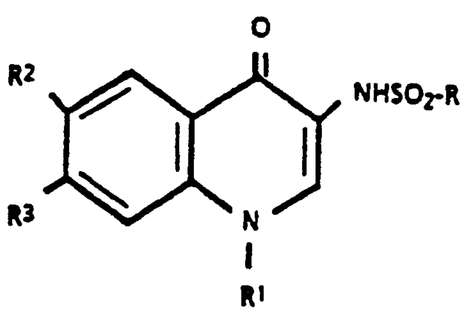

I

wherein R represents an alkyl group containing up to 6 carbon atoms,

$R^1$ represents an alkyl or alkenyl group containing up to 6 carbon atoms,

$R^2$ and $R^3$ singly each represents hydrogen, halogen (preferably fluoro or chloro), methoxy or trifluoromethyl group, with the proviso that at least one of $R^2$ and $R^3$ is hydrogen, and pharmaceutically acceptable salts thereof. Halogen substituents also include bromo and iodo.

The compounds of general formula I, wherein R and $R^1$ represent a methyl group, $R^2$ represents hydrogen or fluoro and $R^3$ represents hydrogen, fluoro or chloro, are prefered.

Preferred compounds are 1-methyl-3-methylsulfonylamino-4(1H) quinolone, 1-methyl-3-methylsulfonylamino-6-fluoro-4(1H)quinolone, 1-methyl-3-methylsulfonylamino-7-chloro-4(1H)quinolone and 1-methyl-3-methylsulfonylamino-7-fluoro-4(1H)quinolone.

According to a feature of the present invention the 4(1H)quinolone derivatives of general formula I may be prepared from the corresponding 3-amino-4(1H)quinolone of the general formula II:

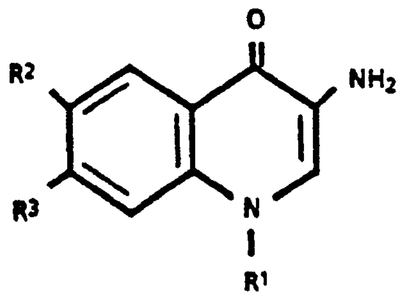

II

(wherein $R^1$, $R^2$, and $R^3$ are as hereinbefore defined) by a process which comprises the condensation with an acid of the general formula III:

$HO_3S$ - R III

or a reactive derivative thereof (wherein R is as hereinbefore defined). The reactive derivative of the said acid may be, for example, a halide (preferably chloride), or the anhydride which are commercially available. The 3-amino-4(1H)quinolones of the general formula II can be prepared by reduction of the corresponding 3-nitro-4(1H)quinolone (T. Itai et al. Chemical Abstracts Vol. 71, 113122 a (1969)).

The reaction of II with the reactive derivative of III is preferably carried out in the presence of an inert organic solvent, for example, tetrahydrofuran, N,N-dimethylformamide, dioxan, toluene, benzene or methylene chloride. The reaction is preferably performed at a temperature between -5° and 120°C.

The 4(1H)quinolones of general formula I may also be prepared according to the invention, by the direct reaction of an acid of general formula III with a 3-amino-4(1H)quinolones of general formula I. The reaction is preferably performed in the presence of an appropriate dehydrating agent. Such agents include, N,N-dicyclohexyl-carbodiimide, carbonyl diimidazol, sulphur trioxide in dimethyl sulphoxide, acetone dimethyl acetal or a polymeric dehydrating agent. The reaction can be carried out in an inert organic solvent, e.g. methylene chloride, acetone, N,N-dimethylformamide, pyridine, ethyl acetate or tetrahydrofuran. Preferably the direct reaction is performed at a temperature between 20° and 110°C.

The 4(1H)quinolones of general formula I can be converted by methods known per se into acid addition salts, for example by reaction of the basic compound with acids in appropriate solvents, for example alcohols, dialkyl ketones or ethers. Suitable acid addition salts are those derivated from inorganic acids, for example the hydrochlorides and sulphates.

The 4(1H)quinolones of general formula I also may be converted into pharmacologically-acceptable salts with an alkali metal hydroxide using, for example, water, methanol, ethanol as a solvent at a temperature between 40° and 100°C. Alkali metals include sodium and potassium.

The experiments with usual test animals were conducted and evaluated in the following manner:

Guinea-pig isolated tracheal rings (spontaneaus tone)

Male guinea-pigs (400-500 g) were killed, and their tracheae were removed and cut into pairs of rings. The rings were then mounted in organ baths containing Krebs-Henseleit solution at 37°C and oxygenated with 95% $CO_2$ + 5% $O_2$ under 1 g resting tension (Allen et al., Br.J.Pharmacol.(1986),89,395). After a 1 hour stabilization period, responses to isoprenaline $10^{-6}$ M were obtained. Tissues were then washed and after a period for restabilization (20 min) the effects of test drugs and flosequinan were investigated by adding cumulative concentrations of these to the baths.

Responses were measured as the percentage of the relaxation response to isoprenaline $10^{-6}$ M. The concentration producing a 50% relaxation taking isoprenaline responses as 100% ($EC_{50}$) was calculated.

Guinea-pig isolated right ventricular strips

Male guinea-pigs of weight 400-500 g were killed, their hearts were removed and 2 ventricular preparations (ca 1 cm x 1 mm) cut from the right bide. There were then mounted in organ baths containing modified Krebs-Henseleit solution at 37°C and continually aereated with 95% $CO_2$ + 5% $O_2$. The preparations were placed under 1 g resting tension and electrically stimulated to contract at 1 Hz whilst force of contraction was measured using an isometric force transducer (Gristwood and Owen, Br.J.Pharmacol. (1985),87,91P). Preparations were equilibrated for 60 min during which they were washed every 15 min with fresh buffer. Flosequinan and test compounds were added to the organ baths from 10-6 to 10-4 M concentrations and responses measured as percentage increases in developed tension.

Vasodilator activity in anaesthetized rats

Male Wistar rats of weight range 300-400 g were anaesthetized with pentobarbital (60 mg/kg i.p.). The trachea was canulated to permit artificial respiration. A carotid artery and a jugular vein were cannulated to permit recording of mean arterial blood pressure and i.v. administration of drugs respectively.

Following a period of stabilisation (20 min), flosequinan or test drugs were administered by bolus injection and falls in mean blood pressure recorded. Responses were measured as the percentage fall in blood pressure over control, calculating the dose that produced an effect of 30% ($ED_{30}$).

Conscious renal hypertensive beagle dogs

Male beagle dogs were made hypertensive by the method of Page (J.Amer.Med.Assoc.(1939),113,2046). Two months later, these animals were prepared with exteriorized carotid loops (Gristwood et al., Br.J.Pharmacol. (1988),93,893) three weeks before using for experimentation. Animals were fasted 17 hours before administration of flosequinan or test compounds orally. Animals were then placed in restraints and allowed to settle for 1 hour. Mean blood pressure (MBP) was measured by puncturing the carotid loop with a 18G needle attached to a pressure transducer. Drugs (suspended in 1% methylcellulose + 0.05% Tween 80) were given orally by gavage at different doses, and mean blood pressure was measured over 4 hours using a polygraph. Drug responses were measured by calculating the area under the curve of percentage fall in mean blood pressure and time (units= % fall in MBP x hours) (Allen et al., J.Clin.Pharmacol.(1982),1S-23S).

Toxicity

Acute toxicity of each compound was determined in groups of 5 male Swiss mice per dose. Administration of flosequinan and test compounds was made by the intraperitoneal route up the dose of 300 mg/kg. After 48 hours the number of deaths was noted and the dose producing 100% of deaths ($LD_{100}$) was calculated.

**TABLE 1. COMPARATIVE RESULTS FOR FLOSEQUINAN AND COMPOUNDS OF FORMULA I**

| COMPOUND* | TRACHEAL RING EC50 (M) | VENTRICULAR STRIP % at 10-5 M | MAN BP IN THE ANAESTH. RAT ED30 (mg/kg iv) | CONSC.HYPERT. DOG AUC (0-4h) at mg/kg po | LD100 IN MICE mg/kg ip |
|---|---|---|---|---|---|
| FLOSEQUINAN | [illegible].00 | 75 (10-4M) | 0.20 | 41 | 300 |
| 1 | 0.24 | 82 | 0.02 | 54 (1 mg/kg) | >300 |
| 3 | 0.60 | 53 | 0.06 | 51 (1 mg/kg) | >300 |
| 4 | 0.50 | 55 (10-6M) | 0.01 | 68 | >300 |
| 5 | 0.42 | 35 (10-6M) | 0.03 | Not Tested | >300 |
| 6 | 0.36 | 86 | 0.01 | 65 | >300 |
| 11 | 1.60 | 52 | 0.05 | Not Tested | >300 |

(*) See structures in Table 2

Table 1 shows that the compounds of formula I are more potent than flosequinan, regarding their effects on guinea-pig tracheal rings (spontaneous tone), and on guinea-pig right ventricular strips where all the compounds were from 3 to 10 times more potent than flosequinan. On mean blood pressure in anaesthetized rats, the title compounds administered intravenously produced an important decrease being, at least, 3 times more potent than flosequinan.

The title compounds were less toxic than flosequinan following i.p. administration to mice and also more active in the conscious hypertensive dog as assessed by the area under the curve, indicating greater potency and duration of action.

Therefore, compounds of the present invention are active agents for treatment of cardiovascular and respiratory disorders such as congestive heart failure, hypertension and asthma.

Thus, the present invention provides 4(1H) quinolone derivatives of the formula I and pharmaceuitcally acceptable salts thereof, and pharmaceutical compositions comprising such derivatives and salts thereof, for use in the treatment or therapy of the human or animal body.

Accordingly, the 4(1H)quinolone derivatives of the formula I and pharmaceutically acceptable salts thereof, and pharmaceutical compositions comprising such derivatives and salts thereof may be used in a method of treatment of cardiovascular and respiratory disorders of the human or animal body which comprises administering to a recipient in need of such therapy an effective amount of said derivatives or salts thereof or said compositions.

The present invention also provides pharmaceutical compositions which comprise, as active ingredient, at least one compound of general formula I, or a pharmacologically acceptable salt in association with a pharmaceutically acceptable carrier or diluent. The active ingredient may comprise 0.001% to 99% by weight, preferably 0.01% to 90% by weight of the composition depending upon the nature of the formulation and whether further dilution is to be made prior to application. Preferably the compositions are made up in a form suitable for oral, topical, percutaneous or parenteral administration.

The pharmaceutically acceptable carriers or diluents which are admixed with the active compound, or compounds or salts of such compounds, to form the compositions of this invention are well known per se and the actual excipients used depend inter alia on the intended method of administering the compositions. Compositions of this invention are preferably adapted for administration per os. In this case, the composition for oral administration may take the form of tablets, capsules, or liquid preparations, such as mixtures, elixirs, syrups or suspensions, all containing one or more compounds of the invention; such preparations may be made by methods well known in the art.

The diluents which may be used in the preparation of the compositions include those liquid and solid diluents which are compatible with the active ingredient, together with colouring or flavouring agents, if desired. Tablets or capsules may conveniently contain between 1 and 50 mg of active ingredient or the equivalent amount of a salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in water or an appropriate parenteral injection fluid.

Effective doses are normally in the range or 5-100 mg of active ingredient per day.

The following Examples illustrate the preparation of compounds of the present invention.

## EXAMPLE 1

To a solution of 1-methyl-3-amino-4(1H)quinolone (2.5 g; 0.0144 moles) in tetrahydrofuran (80 ml) and triethylamine (1.5 g; 0.0148 moles), another solution of methansulfonyl chloride(1.8 g; 0.0157 moles) in tetrahydrofuran (20 ml) was slowly added. The resulting mixture was stirred at room temperature for 2 hours and then heated at 50°C for a further 2 hours. After removing the solvent in vacuo, the residue was treated with water and ethyl acetate, and stirred for some minutes. The organic solution was decanted, dried ($Na_2SO_4$), decolorized, and the solvent removed in vacuo to give 1-methyl-3-methylsulfonamido-4(1H) quinolone (2.5 g; 69.4%). Melting point: 219-220°C (after recrystallization from ethanol).

The 4(1H)quinolone derivatives of general formula I included in Table 2 were prepared according to the process disclosed in this example 1.

TABLE 2

| Compound N° | R | $R^1$ | $R^2$ | $R^3$ | Melting Point °C |
|---|---|---|---|---|---|
| 1 | $CH_3$ | $CH_3$ | H | H | 219-220 |
| 2 | " | " | Cl | " | 265-267 |
| 3 | " | " | F | " | 248-249 (d) |
| 4 | " | " | H | Cl | 254-255 |
| 5 | " | " | " | $CF_3$ | 230-231 |
| 6 | " | " | " | F | 262-263 |
| 7 | " | " | " | $OCH_3$ | 272-274 |
| 8 | " | $C_2H_5$ | " | H | 204-205 |
| 9 | " | $C_3H_7$ | " | " | 232-234 |
| 10 | " | Allyl | " | " | 195-197 |
| 11 | " | $CH_3$ | $OCH_3$ | " | 233-234 |
| 12 | $C_2H_5$ | " | H | " | 194-196 |

EXAMPLE 2

To a suspension of 1-methyl-3-methylsulfonylamino-7-chloro-4(1H) quinolone (1.0 g; 0.0033 moles) in water (10 ml), a solution of sodium hydroxide (0.14 g; 0.0035 moles) in water (10 ml) was added. The resulting mixture was stirred until solution and the solvent removed under reduced pressure to dryness. The residue was recrystallized from a mixture of methanol-water to give 1-methyl-3-methylsulphonylamino-7-chloro-4(1H)quinolone sodium salt, melting point: 308-310°C (d).

EXAMPLE 3

1-methyl-3-methylsulfonylamino-7-chloro-4(1H)quinolone (1.0 g; 0.0035 moles) was suspended in a mixture of concentrated hydrochloric acid (20 ml) and methanol (20 ml). The resulting suspension was heated until solution and then cooled, when 1-methyl-3-methylsulfonylamino-7-chloro-4(1H)quinolone hydrochloride crystallized, melting point: 250-253°C (d).

EXAMPLE 4

100.000 capsules each containing 10 mg of 1-methyl-3-methylsulfonylamino-6-fluoro-4(1H)quinolone (active ingredient) were prepared from the following formulation:

| | |
|---|---|
| 1-methyl-3-methylsulfonylamino-6-fluoro-4(1H)quinolone | 1 kg |
| Lactose monohydrate | 11.7 kg |
| Corn starch | 1 kg |
| Colloidal silicon dioxide | 0.1 kg |
| Magnesium stearate | 0.2 kg |

Procedure

The above ingredients were sieved through a 60-mesh sieve, then mixed in a suitable mixer and filled into 100.000 gelatine capsules.

EXAMPLE 5

1.000 bottles of suspension (capacity 150 ml) each containing 150 mg of 1-methyl-3-methylsulfonylamino-7-chloro-4(1H)quinolone were prepared as follows:

| | |
|---|---|
| 1-methyl-3-methylsulfonylamino-7-chloro-4(1H)quinolone | 150 g |
| microcrystalline cellulose | 1,500 g |
| sodium carboxymethylcellulose | 900 g |
| 70% sorbitol aqueous solution | 33,000 g |
| glycerine | 4,500 g |
| polysorbate 80 | 400 g |
| sodium methyl p-hydroxybenzoate | 240 g |
| sodium propyl p-hydroxybenzoate | 60 g |
| anti-foam silicone | 150 g |
| sodium saccharin | 300 g |
| flavouring | q.s. |
| demineralised water q.s. | 150 litres |

Procedure

To a solution of the sodium methyl p-hydroxybenzoate, sodium propyl p-hydroxybenzoate and sodium saccharin in 30 litres of demineralised water, a wetmilled suspension of the sodium carboxymethylcellulose in glycerine was added. After stirring for 1 hour, a suspension of the microcrystalline cellulose in 60 litres of demineralised water was added and then the sorbitol solution, polysorbate 80, 1-methyl-3-methylsulfonylamino-7-chloro-4(1H)quinolone and flavouring were successively added with stirring. The volume of the mixture was adjusted to 125 litres with demineralised water and milled through a colloidal mill. Antifoam silicone was added and the suspension made up to volume with demineralised water and filled into 150 ml bottles using an appropriate filling machine.

EXAMPLE 6

10.000 suppositories each containing 50 mg of 1-methyl-3-methylsulfonylamino-4(1H)quinolone were prepared as follows:

| | |
|---|---|
| 1-methyl-3-methylsulfonylamino-4(1H)quinolone | 500 g |
| theobroma oil | 19,500 g |

The theobroma oil was melted and the active compound suspended in it. The mixture was then poured into appropriate suppository mould to make 2.0 g suppositories.

**Claims**

1. 4(1H)quinolone derivative of the formula I:

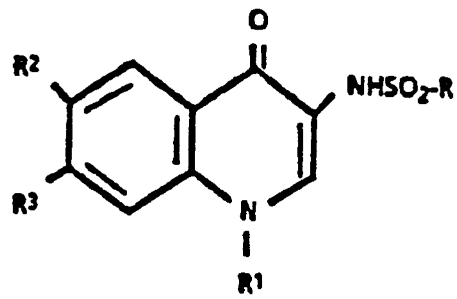

I

wherein R represents an alkyl group containing up to 6 carbon atoms,
$R^1$ represents an alkyl or alkenyl group containing up to 6 carbon atoms,
$R^2$ and $R^3$ singly each represents hydrogen, halogen, methoxy or a trifluoromethyl group, with the proviso that at least one of $R^2$ and $R^3$ is hydrogen, and pharmaceutically acceptable salts thereof.

2. A compound according to claim 1 wherein either $R^2$ is hydrogen and $R^3$ is chloro of fluoro, or $R^3$ is hydrogen and $R^2$ is chloro or fluoro.

3. A compound according to claim 1 or 2 wherein R and $R^1$ represent a methyl group, $R^2$ represents hydrogen or fluoro and $R^3$ represents hydrogen, fluoro or chloro.

4. 1-methyl-3-methylsulfonylamino-4(1H) quinolone.

5. 1-methyl-3-methylsulfonylamino-6-fluoro-4(1H)quinolone.

6. 1-methyl-3-methylsulfonylamino-7-chloro-4(1H)quinolone.

7. 1-methyl-3-methylsulfonylamino-7-fluoro-4(1H)quinolone.

8. A pharmaceutical composition comprising a compound according to any one of claims 1 to 7 together with a pharmaceutically acceptable carrier or diluent.

9. A process for the preparation of 4(1H)quinolone derivatives of general formula I:

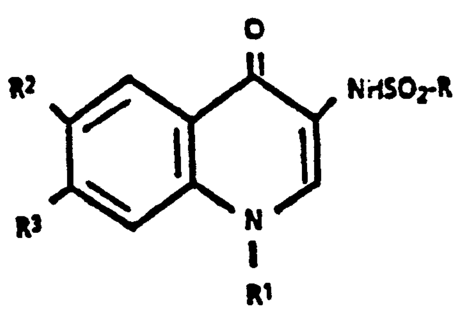

I

wherein R represents an alkyl group containing up to 6 carbon atoms,
$R^1$ represents an alkyl or alkenyl group containing up to 6 carbon atoms,
$R^2$ and $R^3$ singly each represents hydrogen, halogen, methoxy or a trifluoromethyl group, with the proviso that at least one of $R^2$ and $R^3$ is hydrogen, and pharmaceutically acceptable salts thereof, which comprises the condensation of a 3-amino-4(1H)quinolone of the general formula II:

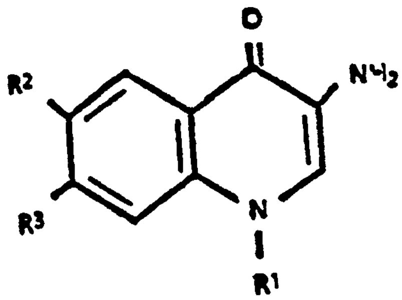

II

(wherein $R^1$, $R^2$, and $R^3$ are as hereinbefore defined) with an acid of the general formula III:

$HO_3S$ - R III

or a reactive derivative thereof (wherein R is as hereinbefore defined).

10. A process according to claim 9 which comprises the direct reaction of the acid of formula III with the 3-amino-4(1H)quinolone of formula II in the presence a dehydrating agent.

11. A compound according to any one of claims 1 to 7 or a composition according to claim 8 for use in a method of treatment or therapy of the human or animal body.

**Claims for the following Contracting States: ES & GR**

1. A process for the preparation of 4(1H)quinolone derivatives of general formula I:

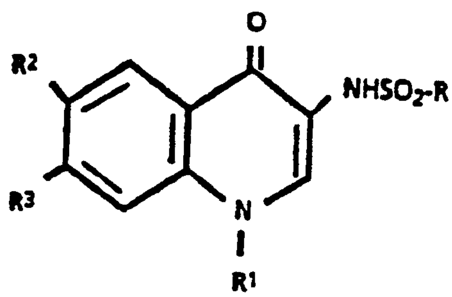

I

wherein R represents an alkyl group containing up to 6 carbon atoms,
$R^1$ represents an alkyl or alkenyl group containing up to 6 carbon atoms,
$R^2$ and $R^3$ singly each represents hydrogen, halogen, methoxy or a trifluoromethyl group, with the proviso that at least one of $R^2$ and $R^3$ is hydrogen, and pharmaceutically acceptable salts thereof, which comprises the condensation of a 3-amino-4(1H)quinolone of the general formula II:

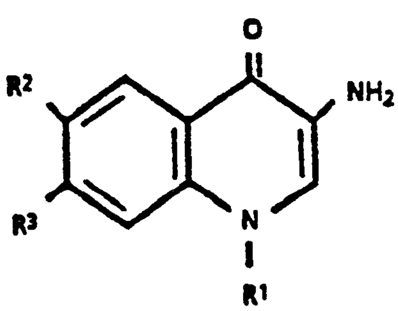

II

(wherein $R^1$, $R^2$, and $R^3$ are as hereinbefore defined) with an acid of the general formula III:

$HO_3S$ - R III

or a reactive derivative thereof (wherein R is as hereinbefore defined).

2. A process according to claim 1 which comprises the direct reaction of the acid of formula III with the 3-amino-4(1H)quinolone of formula II in the presence a dehydrating agent.

3. A process according to claim 1 or 2 wherein either $R^2$ is hydrogen and $R^3$ is chloro of fluoro, or $R^3$ is hydrogen and $R^2$ is chloro or fluoro.

4. A process according to any one of claims 1 to 3 wherein R and $R^1$ represent a methyl group, $R^2$ represents hydrogen or fluoro and $R^3$ represents hydrogen, fluoro or chloro.

5. A process according to any one of claims 1 to 4 wherein 1-methyl-3-methylsulfonylamino-4(1H) quinolone is prepared.

6. A process according to any one of claims 1 to 4 wherein 1-methyl-3-methylsulfonylamino-6-fluoro-4(1H)quinolone is prepared.

7. A process according to any one of claims 1 to 4 wherein 1-methyl-3-methylsulfonylamino-7-chloro-4(1H)quinolone is prepared.

8. A process according to any one of claims 1 to 4 wherein 1-methyl-3-methylsulfonylamino-7-fluoro-4(1H)quinolone is prepared.

9. Process for producing a pharmaceutical composition comprising as active ingredient a 4(1)H quinolone derivative of formula I which comprises mixing a compound according to any one of claims 1 to 8 with a pharmaceutically acceptable carrier or diluent.

European Patent Office

# EUROPEAN SEARCH REPORT

Application Number

EP 91 50 0018

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | JOURNAL OF HETEROCYCLIC CHEMISTRY, vol. 23, 1986, pages 1801-1804, Tampa, US; R. KRISHNAN et al.: "Synthesis of aminoquinolone derivatives" * Page 1802, compound 11 * ----- | 1 | C 07 D 215/38<br>A 61 K 31/47 |

TECHNICAL FIELDS SEARCHED (Int. Cl.5)

C 07 D 215/00

The present search report has been drawn up for all claims

| Place of search | Date of completion of the search | Examiner |
|---|---|---|
| THE HAGUE | 15-05-1991 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)